# EUROPEAN PATENT SPECIFICATION

(11) **EP 3 741 741 B1**
(45) Date of publication and mention of the grant of the patent: **22.06.2022**
(21) Application number: 19741627.4
(22) Date of filing: 17.01.2019
(51) Int. Cl.: C07C 51/44, B01D 3/10, B01D 3/14, B01D 3/34, B01D 5/00, C07C 57/07, F28F 9/22, C07C 51/50, B01D 1/06

(54) **METHOD OF PRODUCING (METH)ACRYLIC ACID OR ESTER THEREOF**
VERFAHREN ZUR HERSTELLUNG VON (METH)ACRYLSÄURE ODER DEREN ESTERN
PROCÉDÉ DE PRODUCTION D'UN ACIDE (MÉTH)ACRYLIQUE OU SON ESTER

(30) Priority: 19.01.2018 JP 2018007446; 11.01.2019 JP 2019003553
(43) Date of publication of application: 25.11.2020
(73) Proprietor: Mitsubishi Chemical Corporation, Chiyoda-ku Tokyo 100-8251 (JP)
(72) Inventor: NAKAMURA Masanari, Tokyo 100-8251 (JP); OGAWA Yasushi, Tokyo 100-8251 (JP)
(74) Representative: Hoffmann Eitle
(86) International application number: PCT/JP2019/001268
(87) International publication number: WO 2019/142862

(56) References cited:
- JP-A- 2003 240 479
- JP-A- 2003 240 479
- JP-A- 2003 240 482
- JP-A- 2005 325 084
- JP-A- 2017 066 130
- JP-A- 2018 008 925
- US-A1- 2005 040 023

## Description

### TECHNICAL FIELD

The present invention which is as defined in the claims relates to a production method of a (meth)acrylic acid or an ester thereof (hereinafter, sometimes referred to as "(meth)acrylic acids"). More specifically, the present invention relates to a production method of (meth)acrylic acids, including a step of, under a reduced pressure created by a pressure reducing device, forming a distillation gas from a process liquid containing (meth)acrylic acids and allowing the distillation gas to turn into a condensate through a vertical multi-tube heat exchanger, wherein continuous production of (meth)acrylic acids is stably performed for a long period of time with preventing production and accumulation of a polymerization product in a pressure reduction system of the vertical multi-tube heat exchanger.

In this connection, the (meth)acrylic acid as used in the present description is a collective term for an acrylic acid and a methacrylic acid and may be either one or both thereof. In addition, the (meth)acrylic acids may be only either one of a (meth)acrylic acid and a (meth)acrylic acid ester or may contain both.

### BACKGROUND ART

The (meth)acrylic acids are a compound which is very easy to polymerize, and the polymerization is promoted in particular under high temperature conditions. If the (meth)acrylic acids are polymerized in the production process thereof, an apparatus and a piping equipment are clogged by a solid matter produced by the polymerization and in the case of serious clogging, an operation cannot be continued. In addition, even if operation stopping due to clogging of the apparatus and the piping equipment is avoided, for example, clogging causes many problems such as an increase in the cleaning frequency of the apparatus and piping equipment during normal operation or in the cleaning load at the time of periodic maintenance.

To cope with these problems, at the time of distillation of (meth)acrylic acids, a polymerization inhibitor is added so as to prevent the polymerization thereof. However, many polymerization inhibitors have a low vapor pressure compared with (meth)acrylic acids, and the polymerization inhibitor added is often scarcely contained in the gas of volatilized (meth)acrylic acids. Even when the polymerization inhibitor is not contained, since the density of (meth)acrylic acids is low in a gas state, it is therefore expected that a polymerization reaction does not substantially occur. However, when the gas is once condensed and turns into a condensate, the condensate has high possibility of polymerization and brings about clogging, *etc.* of the apparatus. The apparatus in which the state above is most likely produced is a condenser for cooling and condensing a distillate gas from a heating distillation apparatus. Accordingly, various studies have heretofore been made so as to prevent (meth)acrylic acids from polymerization in the condenser.

For example, Patent Literature 1 has proposed a method of spraying a liquid containing a polymerization inhibitor on a condensation surface of a vertical multi-tube heat exchanger used as a condenser for cooling and condensing a distillation gas of an easily polymerizable compound.

In addition, Patent Literature 2 has proposed a method where in the distillation and purification of an easily polymerizable compound, at the time of cooling a vapor of the easily polymerizable compound through a vertical multi-tube heat exchanger to form a condensate, by allowing a portion of the obtained condensate to be circulated to the gas introduction side of the vertical multi-tube heat exchanger and uniformly spraying the portion of the obtained condensate on an upper tube plate to wet the inner surface of a condenser tube (heat transfer tube) with the condensate flowing down inside the tube, a distillation gas in an overheated state is prevented from coming into direct contact with the condenser tube and producing a polymerization product. In Patent Literature 2, it is stated that a polymerization inhibitor may be added to the condensate circulated.

The vertical multi-tube heat exchanger includes, as described later, a tubular body, an upper tube plate and a lower tube plate arranged respectively on an upper end side and a lower end side of the tubular body, a plurality of heat transfer tubes erected between the upper tube plate and the lower tube plate, and lid sections arranged respectively on an upper side of the upper tube plate and on a lower side of the lower tube plate. The heat exchanger is configured such that an extraction chamber formed by the lower tube plate and the lid section on the lower side of the lower tube plate is suctioned via a suction pipe by a pressure reducing device, thereby creating a reduced pressure state, and a distillation gas introduced into a receiving chamber formed by the upper tube plate and the lid section on the upper side of the upper tube plate is cooled in the course of passing through the heat transfer tube, turns into a condensate, and is discharged from the bottom of the extraction chamber. In the case of spraying a liquid containing a polymerization inhibitor as in Patent Literatures 1 and 2, the liquid containing a polymerization inhibitor is sprayed to the inside of the receiving chamber into which a distillation gas is introduced. Methods of purification of (meth)acrylic acids or esters thereof involving a vertical multi-tube heat exchanger are further known from US 2005/040023 A1 and JP 2003 240482 A1. These Documents, however, do not disclose a realization of an apparative configuration of vertical multi-tube heat exchangers equipped with baffles and nozzles.

### BACKGROUND ART LITERATURE

### PATENT LITERATURE

Patent Literature 1: JP-A-2000-344688
Patent Literature 2: JP-B-63-11921
Patent Literature 3: US 2005/040023 A1
Patent Literature 4: JP 2003 240482 A1

### SUMMARY OF INVENTION

### TECHNICAL PROBLEM

The conventional vertical multi-tube heat exchanger for cooling and condensing a distillation gas of (meth)acrylic acids has the following problem, and this problem could not be solved even when a liquid containing a polymerization inhibitor is sprayed into a receiving chamber of a vertical multi-tube heat exchanger.

More specifically, a portion of mist of a condensate or an uncondensed gas having reached an inside of an extraction chamber after passing through an inside of a heat transfer tube (hereinafter, referred to a "condensed mist") flows into a suction pipe due to suction created by a pressure reducing device and furthermore, is carried over into the pressure reducing device. The (meth)acrylic acids carried over into a pressure reduction system of the vertical multi-tube heat exchanger, such as suction pipe or pressure reducing device, produce a polymerization product in the pressure reduction system to cause the following problems.
(1) A pressure reduction system such as a suction pipe or a pressure reducing device is clogged with the polymerization product to inhibit pressure reduction by the pressure reducing device or increase the amount of energy for pressure reduction.
(2) In the case of controlling the pressure by an automatic pressure control valve (PCV), production of a polymerization product within PCV makes the pressure control difficult.
(3) A carry over of (meth)acrylic acids into a pressure reduction system causes a decrease in the recovery rate (production efficiency) of (meth)acrylic acids.
(4) A portion of the suction gas in the pressure reduction system is recovered in a hot well tank and thereafter, is processed in a neutralization tank and discharged to the outside of the system or recycled to the process. However, for example, in the production of a (meth)acrylic acid, an acid gets mixed into the hot well tank, and the amount of an alkali agent required for the neutralization increases. In the case of recycling the gas, a (meth)acrylic acid can be recovered without using an alkali agent necessary for neutralization.
(5) A frequency of cleaning for preventing a polymerization product from inhibiting the operation of a pressure reduction system increases.

An object of the present invention is to solve these conventional problems and provide a production method of (meth)acrylic acids, including a step of, under a reduced pressure created by a pressure reducing device, forming a distillation gas from a process liquid containing (meth)acrylic acids and allowing the distillation gas to turn into a condensate through a vertical multi-tube heat exchanger, wherein continuous production of (meth)acrylic acids is stably performed for a long period of time with preventing production and accumulation of a polymerization product in a pressure reduction system of the vertical multi-tube heat exchanger.

### SOLUTION TO PROBLEM

As a result of many intensive studies to solve the problems above, the inventors of the present invention have found that when a vertical multi-tube heat exchanger having a specific configuration of disposing a baffle plate in an extraction chamber is used as the vertical multi-tube heat exchanger for condensing a distillation gas of (meth)acrylic acids, *etc.* and a particle diameter of drops of a liquid containing a polymerization inhibitor sprayed into a receiving chamber of the vertical multi-tube heat exchanger and a gas flow velocity between an inner wall of a lid section near a junction port of a suction pipe and a baffle plate are set to be appropriate values, the above-described object can be attained.

The present invention has been accomplished based on this finding, and the gist thereof resides in the following.

[1] A production method of a (meth)acrylic acid or an ester thereof, including a step of, under a reduced pressure created by a pressure reducing device, forming a distillation gas from a process liquid containing a (meth)acrylic acid or an ester thereof and allowing the distillation gas to turn into a condensate through a vertical multi-tube heat exchanger, wherein
   the vertical multi-tube heat exchanger has
   a tubular body,
   an upper tube plate and a lower tube plate arranged respectively on an upper end side and a lower end side of the tubular body,
   a plurality of heat transfer tubes erected between the upper tube plate and the lower tube plate,
   an upper lid section provided on an upper side of the upper tube plate,
   a lower lid section provided on a lower side of the lower tube plate,
   a receiving chamber surrounded by the upper tube plate and the upper lid section, an extraction chamber surrounded by the lower tube plate and the lower lid section,
   a junction port provided in the lower lid section, to which a suction pipe connecting the vertical multi-tube heat exchanger and the pressure reducing device is joined, and
   a baffle plate provided in the extraction chamber and having a vertical plate section facing the junction port, and
   the distillation gas and a liquid containing a polymerization inhibitor are introduced into the receiving chamber, the distillation gas is cooled in the course of flowing inside the heat transfer tube to produce a condensate, and the condensate and uncondensed distillation gas flow into the extraction chamber,
   an area of the vertical plate section of the baffle plate is larger than an opening area of the junction port,
   a top plate section is provided to connect the upper edge of the vertical plate section and an inner wall of the lower lid section,
   the uncondensed distillation gas within the extraction chamber flows into a relay space surrounded by the baffle plate and the inner wall of the lower lid section and flows out to the suction pipe from the relay space via the junction port,
   the liquid containing a polymerization inhibitor is sprayed as drops from an atomizing nozzle into the receiving chamber,
   an atomizing nozzle where when water is sprayed from the atomizing nozzle under the same conditions as in the case of spraying the liquid containing a polymerization inhibitor from the atomizing nozzle into the receiving chamber, the Sauter mean diameter of water drops sprayed becomes from 570 to 1,500 µm is used as the atomizing nozzle, and
   an average gas flow velocity of the uncondensed distillation gas flowing into the relay space from the extraction chamber is 15.0 m/s or less.
[2] The production method of a (meth)acrylic acid or an ester thereof according to [1], wherein
   the vertical plate section is provided substantially in parallel to the junction port;
   in the baffle plate,
   a first side plate section connecting one side edge of the vertical plate section and the inner wall of the lower lid section and being joined to one side edge of the top plate section, and
   a second side plate section connecting another side edge of the vertical plate section and the inner wall of the lower lid section and being joined to another side edge of the top plate section, are provided; and
   the baffle plate is configured such that the uncondensed distillation gas flows from the baffle plate's lower surface side into the relay space.
[3] The production method of a (meth)acrylic acid or an ester thereof according to [2], wherein when the junction port is projected in a direction perpendicular to the inner wall of the lower lid section onto the vertical plate section, the shortest distance between an outer circumference of a projected image and an outer circumference of the vertical plate section is 50 mm or more.
[4] The production method of a (meth)acrylic acid or an ester thereof according to [2] or [3], wherein a small opening is provided in at least one of the first side plate section and the second side plate section and the uncondensed distillation gas flows also from the small opening into the relay space.
[5] The production method of a (meth)acrylic acid or an ester thereof according to any one of [1] to [4], wherein an extension strip hanging from the lower edge of the vertical plate section and extending with inclining to approach toward the lower lid section on the lower side of the junction port is provided.
[6] The production method of a (meth)acrylic acid or an ester thereof according to any one of [1] to [5], wherein the baffle plate is configured such that the uncondensed distillation gas flows from the baffle plate's lower surface portion and both side surface portions into the relay space.
[7] The production method of a (meth)acrylic acid or an ester thereof according to any one of [1] to [6], wherein the lower lid section has a substantially vertical surface and the junction port is provided in the substantially vertical surface.
[8] The production method of a (meth)acrylic acid or an ester thereof according to any one of [1] to [7], wherein the pressure reducing device is a steam ejector.
[9] The production method of a (meth)acrylic acid or an ester thereof according to any one of [1] to [8], wherein a downwardly inclined section toward the junction port is provided in the suction pipe.
[10] The production method of a (meth)acrylic acid or an ester thereof according to any one of [1] to [9], wherein the exterior surface of the suction pipe is kept warm with a heat-insulating material.
[11] The production method of a (meth)acrylic acid or an ester thereof according to any one of [1] to [10], wherein the exterior surface of the suction pipe is warmed by steam tracing or electric tracing.

### EFFECTS OF INVENTION

According to the present invention, in a production method of (meth)acrylic acids, including a step of, under a reduced pressure created by a pressure reducing device, forming a distillation gas from a process liquid containing (meth)acrylic acids and allowing the distillation gas to turn into a condensate through a vertical multi-tube heat exchanger, continuous production of (meth)acrylic acids can be stably performed for a long period of time based on the following actions and effects with preventing production and accumulation of a polymerization product in a pressure reduction system of the vertical multi-tube heat exchanger.
(1) Since the pressure reduction system can be prevented from clogging with a polymerization product, the degree of pressure reduction can be more efficiently increased by a pressure reducing device though a suction pipe and at the same time, the amount of energy for pressure reduction can be reduced.
(2) Pressure control by an automatic pressure control valve (PCV) can be smoothly performed.
(3) A recovery rate (production efficiency) of (meth)acrylic acids can be increased by preventing carry over of (meth)acrylic acids into a pressure reduction system.
(4) An amount of an alkali agent required for neutralization of a recovered liquid in a hot well tank can be decreased.
(5) The operation stopping period or labors for cleaning can be reduced by decreasing cleaning frequency.

### BRIEF DESCRIPTION OF DRAWINGS

[Fig. 1] Fig. 1 is a schematic system diagram illustrating one example of an embodiment of the production method of (meth)acrylic acids according to the present invention.
[Fig. 2] Fig. 2 is a schematic system diagram illustrating the vertical multi-tube heat exchanger used in the present invention and its pressure reduction system.
[Fig. 3] Fig. 3 is a perspective diagram illustrating one embodiment of the baffle plate of the vertical multi-tube heat exchanger of Fig. 2.
[Fig. 4] Fig. 4 is a perspective diagram of one embodiment of the baffle plate.
[Fig. 5] Fig. 5 is an exploded perspective diagram of one embodiment of the baffle plate.
[Fig. 6] Fig. 6 is a perspective diagram of one embodiment of the baffle plate.
[Fig. 7] Fig. 7 is an exploded perspective diagram of one embodiment of the baffle plate.
[Fig. 8] Fig. 8 is a right-side view of the baffle plate of Fig. 6.
[Fig. 9] Fig. 9 is a left-side view of the baffle plate of Fig. 6.
[Fig. 10] Fig. 10 is a bottom view of the baffle plate of Fig. 6.
[Fig. 11] Fig. 11 is a diagram explaining a dimension of the baffle plate of Fig. 6.

### DESCRIPTION OF EMBODIMENTS

The embodiments of the production method of (meth)acrylic acids of the present invention are described in detail below by referring to the drawings. However, the present invention is not limited by any means to the contents in the following description and can be implemented by making various changes within the scope of the gist of the present invention.

In the following, although an embodiment of condensing a distillation gas of acrylic acid is described, the present invention is not limited to an acrylic acid and can be widely applied to the condensation of a distillation gas of (meth)acrylic acids.

Furthermore, in the following, numerical values provided to the dimension of each section of the vertical multi-tube heat exchanger are exemplary only as a vertical multi-tube heat exchanger used in general-purpose commercial facilities, and the dimension of each section of the vertical multi-tube heat exchanger according to the present invention is not limited by any means to those described below.

In the present invention, the "substantially in parallel" encompasses a ±10° tilt range with respect to parallelism, and the "substantially vertical surface" encompasses a ±10° tilt range with respect to a vertical surface.

Furthermore, in the present invention, "upper" means a direction opposite to the gravity direction, and "lower" means the gravity direction.

The present invention is a production method of a (meth)acrylic acid or an ester thereof, including a step of, under a reduced pressure created by a pressure reducing device, forming a distillation gas from a process liquid containing a (meth)acrylic acid or an ester thereof and allowing the distillation gas to turn into a condensate through a vertical multi-tube heat exchanger.

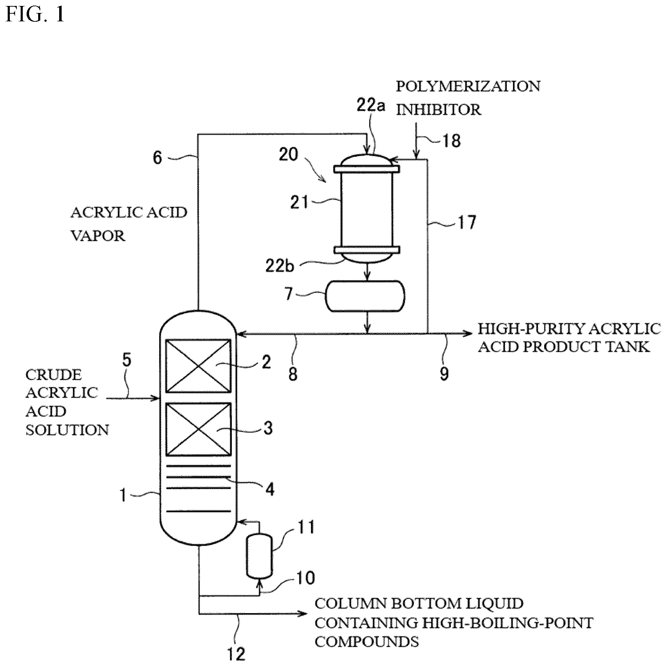

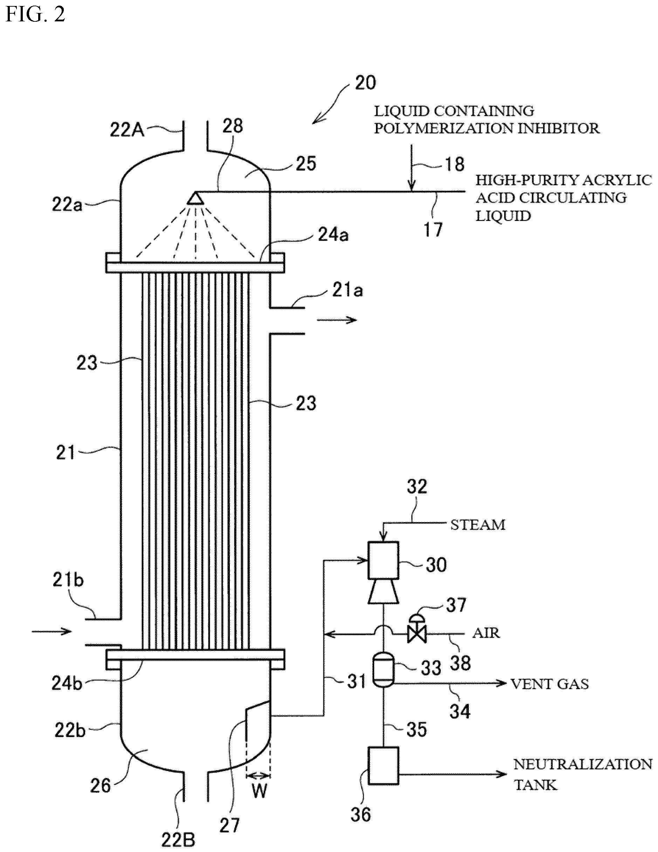

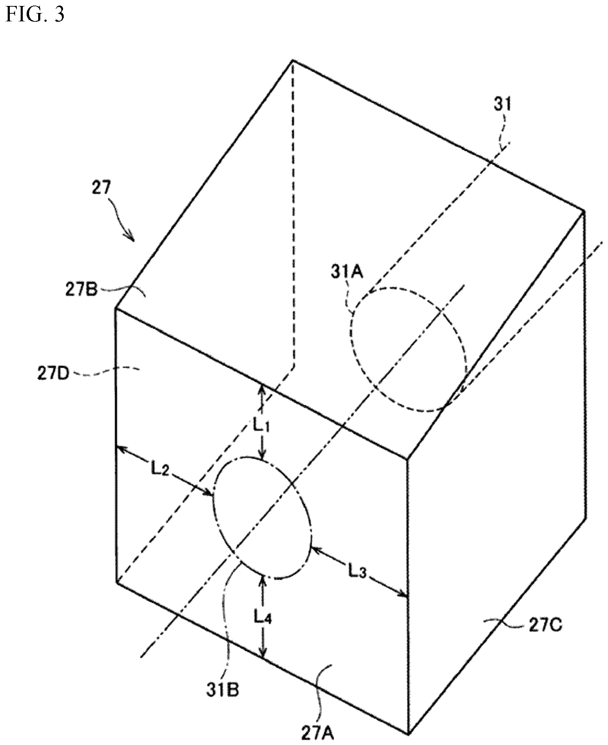

Fig. 1 is a schematic system diagram illustrating one example of the embodiment of the production method of (meth)acrylic acids according to the present invention. Fig. 2 is a schematic system diagram illustrating the vertical multi-tube heat exchanger used in the present invention and its pressure reduction system. Fig. 3 is a perspective diagram illustrating one embodiment of the baffle plate of the vertical multi-tube heat exchanger of Fig. 2.

In this connection, Fig. 1 illustrates a case where a high-temperature distillation gas of crude acrylic acid from a distillation column 1 is directly introduced into the vertical multitube heat exchanger 20 and condensed. However, the present invention is not limited by any means to such an embodiment and can also be applied, for example, to an embodiment where a relatively low-temperature uncondensed gas obtained after partially condensing a high-temperature distillation gas from a distillation column 1 by a heat exchanger (condenser) provided in a stage prior to the vertical multi-tube heat exchanger 20 is further condensed by the vertical multi-tube heat exchanger 20.

Fig. 1 illustrates, in the process of producing an acrylic acid, a high-purity acrylic acid distillation step of feeding a crude acrylic acid obtained through a reaction step and a purification step of an acrylic acid to a distillation column 1 and separating by distillation into an acrylic acid and a high-boiling-point impurity. In Fig. 1, the distillation column 1 includes, as inserts, a regular filler 2 in a concentration section, an irregular filler 3 in an upper recovery section, and under the irregular filler 3, plates 4 having perforated plates without weir. A crude acrylic acid solution containing an extraction solvent is fed to the distillation column 1 by a feed line 5. An acrylic acid vapor separated by a column top gas line 6 is cooled and condensed by the vertical multi-tube heat exchanger 20 and recovered in a reflux tank 7. A portion of the recovered acrylic acid is circulated to a column top section of the distillation column 1 by a reflux line 8, another portion is circulated as a spray liquid to an upper part of the vertical multi-tube heat exchanger 20, and the remainder is sent to a high-purity acrylic acid product tank (not illustrated) by a remainder line 9. The column bottom liquid of the distillation column 1 is heated in a reboiler 11 after passing through a circulation line 10 and then circulated to the distillation column 1. A portion of the column bottom liquid containing high-boiling-point compounds is recovered from a withdrawal line 12.

The outer peripheries of the distillation column 1 and the column top gas line 6 are warmed by means of an electric heater or a steam pipe (steam tracing) and further kept warm with a heat-insulating material so as to prevent the withdrawn distillation gas from condensing halfway. In addition, the column top gas line 6 is downwardly inclined toward the distillation column 1 side so that even if a condensate is produced, the condensate cannot stay inside.

A temperature of the distillation gas of crude acrylic acid fed to the vertical multitube heat exchanger 20 is usually on the order of 50 to 110°C. However, as described above, in the case of partially condensing the distillation gas by a heat exchanger (condenser) provided separately in a stage prior to the vertical multi-tube heat exchanger 20, the temperature of the uncondensed gas fed to the vertical multi-tube heat exchanger 20 is approximately from 15 to 50°C.

The vertical multi-tube heat exchanger 20 has a tubular body 21 and upper and lower lid sections 22a and 22b provided at its both ends and has a plurality of heat transfer tubes 23 inside the tubular body 21.

More specifically, as illustrated in Fig. 2, the vertical multi-tube heat exchanger 20 has a tubular body 21 disposed such that the axial direction runs in the vertical direction, upper and lower tube plates 24a and 24b arranged respectively on the upper end side and the lower end side of the tubular body 21 such that the plate surface runs in the horizontal direction, a plurality of heat transfer tubes 23 erected in the vertical direction between the upper tube plate 24a and the lower tube plate 24b by fixing respective tube end parts to the upper tube plate 24a and the lower tube plate 24b, and domed upper and lower lid sections 22a and 22b arranged respectively on the upper side of the upper tube plate 24a and on the lower side of the lower tube plate 24b, and a receiving chamber 25 and an extraction chamber 26 are formed respectively in a space between the upper tube plate 24a and the upper lid section 22a and in a space between the lower tube plate 24b and the lower lid section 22b.

As illustrated in Fig. 2, a junction port 31A of a suction pipe 31 is provided in the side wall section of the lower lid section 22b. A suction pipe 31 coupled with a steam ejector 30 serving as a pressure reducing device is connected to the junction port 31A and is configured to reduce the pressure in the extraction chamber 26 by a suction effect of steam from a steam pipe 32. Agas suctioned by the steam ejector 30 is cooled in a condenser 33, and a vent gas is discharged through a discharge pipe 34 to the outside of the system.

On the other hand, a condensate cooled and liquefied in a condenser 33 is recovered and accumulated in a hot well tank 36 through a pipe 35, then fed to a neutralization tank (not illustrated), neutralized and thereafter, discharged to the outside of the system. Air is introduced into the suction pipe 31 through a pipe 38 via a pressure control valve 37, and a degree of pressure reduction created by the steam ejector 30 is thereby adjusted. In this connection, the pressure regulation by the pressure control valve 37 includes a case of suctioning air through the pipe 38 as illustrated in Fig 2 and a case of recycling the gas at the steam ejector 30 outlet.

In the extraction chamber 26 of the vertical multi-tube heat exchanger 20, a baffle plate 27 having a vertical plate section 27A facing the junction port 31A of the suction pipe 31 is provided.

An inflow port 21b for a cooling medium (cooling water) is provided in the lower part of the side wall between the upper tube plate 24a and the lower tube plate 24b of the tubular body 21, and an outflow port 21a is provided in the upper part. As a result of suction and pressure reduction created by the steam ejector 30, a distillation gas containing an acrylic acid as a main component, which has been introduced into the receiving chamber 25 through an inflow port 22A provided in the upper part of the upper lid section 22a of the vertical multi-tube heat exchanger 20, is cooled in the course of flowing down inside the heat transfer tube 23 by the cooling medium flowing outside the heat transfer tube 23 to produce a condensate. The condensate is recovered the reflux tank 7 illustrated in Fig. 1 from an outflow port 22B provided in the lower part of the lower lid section 22b through the extraction chamber 26 formed in a space between the lower tube plate 24b and the lower lid section 22b. As described above, a portion of the condensate is circulated to the column top section of the distillation column 1, another portion is circulated to the receiving chamber 25 side of the vertical multi-tube heat exchanger 20 by a circulation line 17, and the remainder is sent to a high-purity acrylic acid product tank (not illustrated) by a remainder line 9. A solution containing a polymerization inhibitor is fed to the circulation line 17 through a polymerization inhibitor feed line 18, and a high-purity acrylic acid circulating liquid containing a polymerization inhibitor (hereinafter, referred to as "liquid containing a polymerization inhibitor") is fed into the receiving chamber 25 of the vertical multi-tube heat exchanger 20.

Although a form of feed of the liquid containing a polymerization inhibitor to the receiving chamber 25 is not particularly limited, the liquid containing a polymerization inhibitor is preferably fed to achieve full contact with a distillation gas introduced into the receiving chamber 25. For example, the liquid containing a polymerization inhibitor is preferably sprayed like mist throughout the interior of the receiving chamber 25 from an atomizing nozzle 28 inserted into the central part of the receiving chamber 25 from the upper lid section 22a so as to increase a contact area of the liquid containing a polymerization inhibitor with the distillation gas as much as possible and also to spread the liquid containing a polymerization inhibitor all over the upper tube plate 24a.

An uncondensed distillation gas that has not been condensed in the course of flowing down inside the heat transfer tube 23 flows into the extraction chamber 26, flows into the later-described relay space 27T, and flows out of the relay space 27 T to the suction pipe 31 via the junction port 31A.

Fig. 3 is a perspective diagram illustrating one embodiment of the baffle plate 27 provided in the extraction chamber 26 of the vertical multi-tube heat exchanger 20 of Fig. 2. The baffle plate 27 has a vertical plate section 27A and a top plate section 27B and preferably has a first side plate section 27C and a second side plate section 27D.

The vertical plate section 27A is preferably provided such that the plate surface faces the junction port 31A of the suction pipe 31 bored through the lower lid section 22b (namely, the opening section of the suction pipe 31 toward the lower lid section 22b) and runs substantially in parallel to the junction port 31A with a predetermined spacing.

The top plate section 27B is provided such that the base end side combines with the inner wall of the lower lid section 22b and the distal end side combines with the upper end of the vertical plate section 27A.

The first side plate section 27C is provided such that one side edge part combines with one side edge part of the vertical plate section 27A, another side edge part combines with the inner wall of the lower lid section 22b, and the upper edge part combines with one side edge part of the top plate section 27B.

The second side plate section 27D is provided such that one side edge part combines with another side edge part of the vertical plate section 27A, another side edge part combines with the inner wall of the lower lid section 22b, and the upper edge part combines with another side edge part of the top plate section 27B.

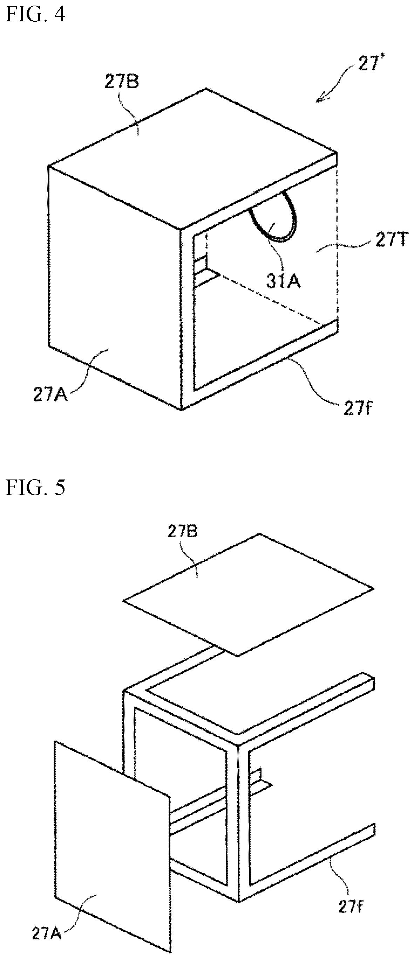

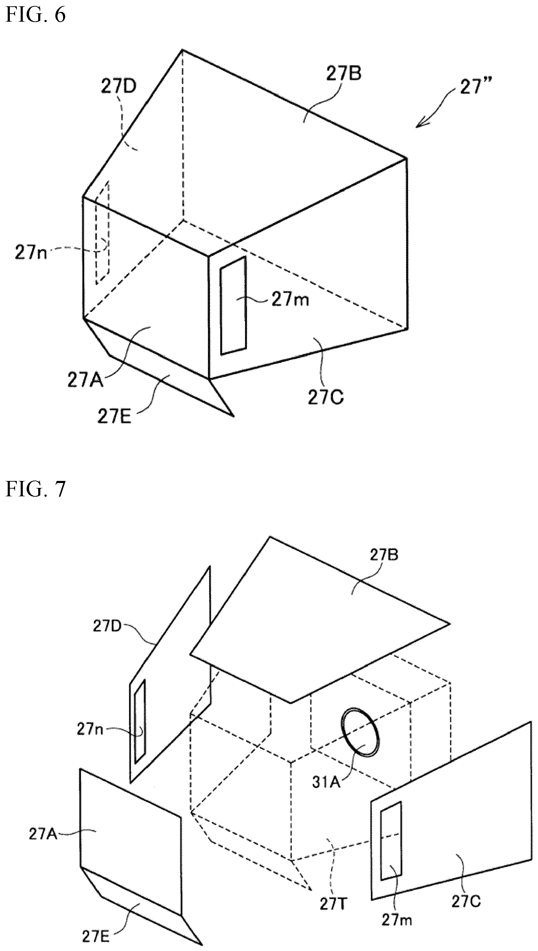

The relay space 27T with the lower surface being opened (Fig. 4, Fig. 7) is formed by the baffle plate 27 and the inner wall of the lower lid section 22b. In this embodiment, the top plate section 27B is an inclined plate downwardly inclining from the base end side toward the distal end side.

In the present invention, an area of the plate surface of the vertical plate section 27A is larger than an opening area of the junction port 31A (corresponding to an opening area of the suction pipe 31). In addition, when the junction port 31A is projected in a direction perpendicular to the inner wall of the lower lid section 22b onto the vertical plate section 27A, a shortest distance between the outer circumference of the projected image (in Fig. 3, the circle 31B denoted by the dashed-dotted line) of the junction port 31A formed in the vertical plate section 27A and the outer circumference of the plate surface of the vertical plate section 27A (hereinafter, this distance is sometimes simply referred to as "dimensional difference L") is preferably 50 mm or more. In other words, all of L₁, L₂, L₃ and L₄ in Fig. 3 are preferably 50 mm or more.

If the dimensional difference L is less than 50 mm, the baffle plate 27 cannot sufficiently prevent the inflow of condensed mist or uncondensed gas into the suction pipe 31, and a polymerization product may be formed within the polymerization system.

From the viewpoint of preventing formation of a polymerization product in the polymerization system, the dimensional difference L is preferably larger and in particular preferably 55 mm or more. On the other hand, if the dimensional difference L is excessively large, condensation of the gas may occur due to a reduction in the gas flow velocity. In addition, in view of restriction on the space size of the extraction chamber 26 of the vertical multi-tube heat exchanger 20, the dimensional difference L is usually 100 mm or less and among others, is preferably 95 mm or less.

In this connection, although in the baffle plate 27 illustrated in Fig. 3, the top plate section 27B is an inclined plate, the top plate section 27B may be provided in a horizontal direction. However, as illustrated in Fig. 3, when the section above is an inclined plate forming such a downward slope that the angle between the top plate section 27B and the vertical plate section 27A is approximately from 100 to 130°, this is advantageous in that condensed mist on the top plate section 27B can smoothly flow down to the outflow port 22B side of the extraction chamber 26.

In the present invention, from the viewpoint of preventing the liquid retention, the junction port 31A of the suction pipe 31 is preferably provided in a substantially vertical direction. The side wall portion of the lower lid section 22b of the vertical multi-tube heat exchanger 20 has a portion forming a substantially vertical surface, and for the reason above, the junction port 31A is preferably provided in this substantially vertical side wall section.

Although Fig. 3 illustrates an example where the vertical plate section 27A of the baffle plate 27 is square-shaped, the vertical plate section of the baffle plate is sufficient if the dimensional difference L is 50 mm or more, and the shape thereof is not limited to a square and may be circle or other shapes.

With respect to the top plate section 27B, its dimension in the width direction is preferably equal to that of the vertical plate section 27A. Usually, the vertical plate section 27A, top plate section 27B, first side plate section 27C and second side plate section 27D of the baffle plate 27 are prepared by processing a plate material composed of a stainless steel (e.g., SUS304, SUS304L, SUS316, SUS316L) and fixed to the side wall section of the lower lid section 22b of the vertical multi-tube heat exchanger 20 by welding, *etc.*

From the viewpoint of more unfailingly preventing inflow of condensed mist or uncondensed gas into the pressure reduction system by providing a baffle plate 27, the baffle plate 27 is preferably provided at such a position that the area of the opening section of the baffle plate 27 (the area of the lower surface potion of the baffle plate 27 in Fig. 3) becomes approximately from 2 to 3 times the area of the junction port 31A (opening area of the suction pipe 31) and the above-described projected image of the junction port 31A in the vertical plate section 27A is formed at the center of the plate surface of the vertical plate section 27A.

In addition, the distance (distance W in Fig. 2) between the vertical plate section 27A of the baffle plate 27 and the junction port 31A is sufficient if it is a distance enabling to obtain the later-described gas flow velocity within the baffle plate, and although it varies depending on, for example, the size of the vertical plate section 27A of the baffle plater 27 or the inner diameter of the vertical multi-tube heat exchanger 20, the distance is usually equal to the diameter of the junction port 31A (the diameter is the bore of the suction pipe 31 and is usually on the order of 100 to 500 mm).

Using a vertical multi-tube heat exchanger configured as above, a liquid containing a polymerization inhibitor is sprayed as drops having a particle diameter within a predetermined range from an atomizing nozzle into the receiving chamber of the vertical multi-tube heat exchanger.

In the present invention, an atomizing nozzle where when water is sprayed from the atomizing nozzle under the same conditions as in the case of spraying a liquid containing a polymerization inhibitor from the atomizing nozzle into the receiving chamber, the Sauter mean diameter of water drops sprayed becomes from 570 to 1,500 µm is used (since the conditions are the same, the atomizing nozzle diameter, spray angle, feed pressure to nozzle, and feed amount are of course the same). In this connection, this average particle diameter of water drops measured using water is substantially the same as the average particle diameter of drops of the liquid containing a polymerization inhibitor which are sprayed from the atomizing nozzle and therefore, the average particle diameter of sprayed water drops measured using water is hereinafter referred to as the particle diameter of sprayed drops of the liquid containing a polymerization inhibitor.

If the particle diameter of sprayed drops of the liquid containing a polymerization inhibitor is less than 570 µm, the liquid drop or the condensed mist or uncondensed gas together with the liquid drop is likely to flow into the suction pipe from the junction port of the suction pipe.

Namely, as a result of many studies to attain the object of the present invention, the present inventors have found that drops of liquid containing a polymerization inhibitor which is sprayed like mist turn into a liquid in the process of flowing down inside the heat transfer tube from the upper tube plate but thereafter, when discharged from the lower tube plate, the liquid again returns to liquid drops having the same particle diameter as the liquid drops sprayed and reaches the extraction chamber. In addition, the present inventors have found that if the particle diameter of the drops of the liquid containing a polymerization inhibitor in the extraction chamber is small, the liquid drops as well as the condensed mist or uncondensed gas entrained with the liquid drop are likely to flow into the suction pipe from the junction port.

From this viewpoint, the particle diameter of sprayed drops of the liquid containing a polymerization inhibitor is preferably larger. However, since it is difficult to excessively increase the particle diameter of liquid drops, the particle diameter of the liquid drops is preferably from 600 to 1,500 µm, particularly on the order of 700 to 1,000 µm.

The liquid containing a polymerization inhibitor can be sprayed as drops having such a particle diameter by selecting and using a commercially available spray nozzle having the capability of spraying liquid drops with such a particle diameter.

Furthermore, in the present invention, it is an essential requirement that an average gas flow velocity (hereinafter, sometimes referred to as "gas flow velocity") of the gas flowing into the relay space 27T from the extraction chamber 26 is 15.0 m/s or less.

The average gas flow velocity is calculated by dividing a gas flow rate by an opening area. The gas flow rate can be calculated using a pressure reducing capacity of a steam ejector, *etc.,* an amount of air fed to a reboiler, *etc.,* a vapor amount calculated from a vapor pressure of an acrylic acid, *etc.* at a pressure and temperature in the extraction chamber, and an amount of outside air leaked into the multi-tube heat exchanger, *etc.* under a reduced pressure.

If the gas flow velocity exceeds 15.0 m/s, condensed mist or uncondensed gas is likely to flow into the suction pipe from the junction port. In order to more unfailingly prevent the condensed mist or uncondensed gas from flowing into the suction pipe, the gas flow velocity is preferably 14.0 m/s or less, and particularly preferably 13.5 m/s or less. However, if the gas flow velocity is too small, the inside of the vertical multi-tube heat exchanger may be unable to create sufficient pressure reduction conditions. Therefore, the lower limit of the gas flow velocity is, usually, preferably 3 m/s or more.

In the case of the baffle plate 27, the gas flow velocity is a value V/A (m/s) obtained by dividing a flow rate V(Nm³/s) of the gas flowing out from the junction port 31A by the area A (m²) of the opening section in the lower part of the baffle plate 27.

The gas flow velocity can be made to fall in the above-described preferable range by determining the size of the opening section in the lower part of the baffle plate 27 according to the gas flow rate.

Although in the embodiment above, the baffle plate 27 has a bottomless box shape in which the upper surface, the front surface (surface in front of the junction port 31A, hereinafter the same) and both of the right and left-side surfaces are closed and the lower part is opened, in the present invention, a baffle plate having other shapes may be used. Figs. 4 and 5 and Figs. 6 to 11 illustrate an example thereof.

The baffle plate 27' of Figs. 4 and 5 has a vertical plate section 27A and a top plate section 27B, and the left-side surface, the right-side surface and the lower part are opened. In this connection, the vertical plate section 27A and the top plate section 27B are fixed to a frame 27f. The gas inside the lower lid section 22b flows out from the junction port 31A through the left-side surface, right-side surface and opening section in the lower part of the baffle plate 27' via the inside of the relay space 27T.

In this case, the opening area of the inflow section into the baffle plate 27' is the total of the area of opening section in the left-side surface, the area of the opening section in the right-side surface, and the area of the opening section in the lower part, of the baffle plate 27'.

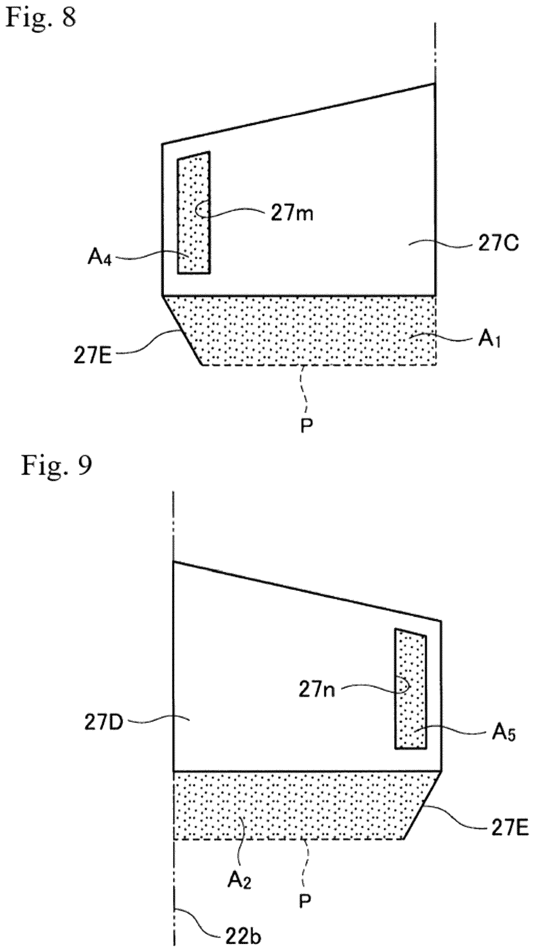

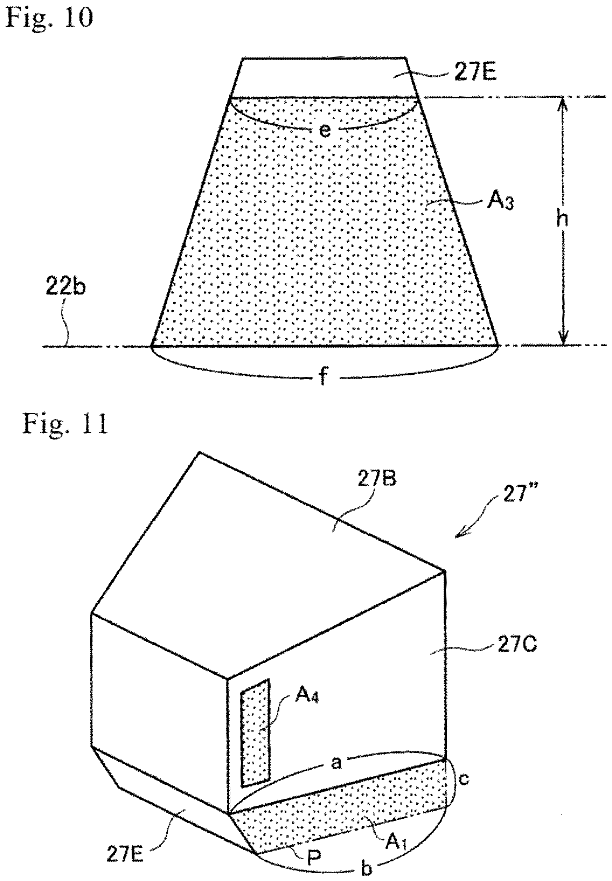

Figs. 6 to 11 illustrate a baffle plate 27". Fig. 6 is a perspective diagram of the baffle plate 27". Fig. 7 is an exploded perspective diagram of the baffle plate 27". Fig. 8 is a right-side view of the baffle plate 27". Fig. 9 is a left-side view of the baffle plate 27". Fig. 10 is a bottom view of the baffle plate 27". Fig. 11 is a diagram explaining a dimension of the baffle plate 27".

The baffle plate 27" has a vertical plate section 27A, a top plate section 27B, a first side plate section 27C, a second side plate section 27D, small openings 27m and 27n each provided in the vertical direction at the front part of at least one of the first side plate section 27C and the second side plate section 27D, and an extension strip 27E hanging from the lower edge of the vertical plate section 27A and extending with inclination toward the lower lid section 22b inner wall on the lower side of the junction port 31A.

The gas inside the lower lid section 22b flows to the junction port 31A from the lower part, small opening 27m and small opening 27n of the baffle plate 27" through the inside of the relay space 27T.

In this case, the opening area of the inflow section into the baffle plate 27" is the total of the areas A1 and A₄ depicted in Figs. 8 and 11, the areas A₂ and A₅ depicted in Fig. 9, and the area A₃ depicted in Fig. 10. A₄ and A₅ are opening areas of the small opening 27m and small opening 27n.

The area A₁ is, as illustrated in Fig. 11, the area of a trapezoid having an upper edge a, a lower edge b, and a height c. The lower edge b is a line of intersection with a horizontal plane P when the first side plate section 27C is extended onto the horizontal plane P.

The baffle plate 27" has a bilaterally symmetrical shape, and the area A₂ is equal to the area A₁.

The area A₃ is the area of a trapezoid having a lower edge e of the extension strip 27E, a line segment f connecting rear ends of the first side plate section 27C and the second side plate 27D, and a distance h between the lower edge e and the line segment f.

Here, in the present invention, the degree of pressure reduction created by a pressure reducing device such as a steam ejector is preferably on the order of 1 to 80 kPaA in terms of the pressure of the reflux tank 7.

As the pressure reducing device, a vacuum pump, *etc.* may be used, other than a steam ejector. However, in view of long-term continuous operation, a steam ejector is preferred.

According to the present invention, the above-described baffle plate configuration, the particle diameter of sprayed drops of the liquid containing a polymerization inhibitor, and the gas flow velocity within the baffle plate are made to fall within the ranges of the present invention, so that inflow of condensed mist or uncondensed gas into the suction pipe can be sufficiently prevented. In this connection, an inclined section having a downward slope (sloping downwardly) toward the junction pipe may be provided in the suction pipe so that when condensed mist or uncondensed gas has flowed into the suction pipe due to some conditional change and turned into a condensate, the condensate within the suction pipe can be caused to flow down and return to the extraction chamber side of the vertical multi-tube heat exchanger.

In addition, the exterior surface of the suction pipe is preferably kept warm with a heat-insulating material or warmed by steam tracing or electric tracing so as to prevent the production and accumulation of a polymerization product within the suction pipe.

As illustrated in Fig. 1, out of the condensate withdrawn from the lower part of the vertical multi-tube heat exchanger, the amount of the high-purity acrylic acid circulating liquid which is circulated to the receiving chamber side of the vertical multi-tube heat exchanger is, in view of the polymerization preventing effect due to circulation of the high-purity acrylic acid circulating liquid and the production efficiency, preferably on the order of 3 to 70 mass% of the condensate withdrawn from the lower part of the vertical multi-tube heat exchanger. In this connection, the temperature of the condensate is, usually, approximately from 20 to 60°C.

However, in the present invention, the liquid containing a polymerization inhibitor introduced into the receiving chamber of the vertical multi-tube heat exchanger may not be a solution formed by circulating a part of the condensate from the lower part of the heat exchanger and may be a solution obtained by adding a polymerization inhibitor to the solution containing high-purity acrylic acid from other systems.

As the polymerization inhibitor, all of the polymerization inhibitors conventionally employed for the production of (meth)acrylic acids can be used, and one member or two or more members of, for example, phenols such as hydroquinone and hydroquinone monomethyl ether, amines such as phenothiazine and diphenylamine, heavy metal salts such as copper dibutyldithiocarbamate and manganese acetate, a nitroso compound, a nitro compound, and aminoxyls such as tetramethylpiperidinoxyl derivative, may be used.

The concentration of the polymerization inhibitor in the liquid containing a polymerization inhibitor is preferably on the order of 10 to 2,000 ppm from the viewpoint of sufficiently obtaining the polymerization preventing effect due to the addition of a polymerization inhibitor and furthermore, preventing the problem in the post process, such as precipitation.

### EXAMPLES

The present invention is described in greater detail below by referring to Examples. However, the present invention is not limited to the following Examples.

### [Example 1]

Crude acrylic acid obtained by using propylene as a raw material through a reaction step of performing a catalytic gas phase oxidation reaction and a purification step was fed to a distillation column 1 illustrated in Fig. 1, and distillation of acrylic acid was continuously performed. A column top pressure of the distillation column 1 and a reflux ratio were set to be 2.4 kPaA and 1.2, respectively, and acrylic acid vapor distilled out of the column top was fed to a vertical multi-tube heat exchanger 20 having a configuration illustrated in Fig. 2.

As the vertical multi-tube heat exchanger 20, a heat exchanger having 225 SUS304-made heat transfer tubes with an inside diameter of 1 inch was used. A feed temperature of cooling water was from 15 to 19°C, and a temperature of a condensed high-purity acrylic acid was from 30 to 33°C. Approximately from 5 to 10 mass% of the condensate was circulated to the receiving chamber 25 side. To the circulating liquid, an acrylic acid solution of hydroquinone monomethyl ether was added as a polymerization inhibitor such that the hydroquinone monomethyl ether concentration becomes about 200 ppm. An atomizing nozzle 28 was provided on the tip of the circulation line in order for the circulating liquid to spread all over the upper tube plate 24a.

As illustrated in Fig. 2, an inside of an extraction chamber 26 of the vertical multitube heat exchanger 20 was suctioned via a suction pipe 31 by a steam ejector 30 so as to cause the degree of pressure reduction in a reflux tank 7 to become 2.4 kPaA.

A side wall section having a junction port 31A of the suction pipe 31 in the lower lid section 22b was provided in the vertical direction, and a baffle plate 27 having the shape illustrated in Fig. 3 was provided to face the junction port 31. A bore of the suction pipe 31 was 150 mm, and an opening area in the lower part of the baffle plate 27 was 39,000 mm².

The baffle plate 27 was made of SUS304, and the specification thereof is as follows.

Top plate section: 260 mm×173 mm, an inclined plate of 120°
Vertical plate section: 260 mm×260 mm, a vertical plate
Distance W between the vertical plate section and the junction port: 150 mm
Side plate section: A trapezoid having an upper edge of 173 mm, a vertical plate section-side side edge of 260 mm, a bottom edge of 150 mm, and a lid section inner wall-side side edge of about 347 mm
Dimensional difference L₁: 55 mm
Dimensional difference L₂: 55 mm
Dimensional difference L₃: 55 mm
Dimensional difference L₄: 55 mm

A nozzle manufactured by Niikura Kogyo Co., Ltd. was used as the atomizing nozzle 28, and drops having a particle diameter of 950 µm of a liquid containing a polymerization inhibitor were sprayed.

The gas flow velocity within the baffle plate was 13.3 m/s

Distillation was performed in this way, as a result, a problem such as production of a polymerization product was not caused in the pressure reduction system of the vertical multi-tube heat exchanger 20 even after the elapse of 120 days from the start of operation, and the operation could be stably continued.

### [Comparative Example 1]

Distillation was performed in the same manner except that in Example 1, the dimensional differences L₁ to L₄, the gas flow velocity within the baffle plate, and the particle diameter of sprayed liquid drops were changed as follows. As a result, the number of days for which the operation can be stably continued was 10 days.

Dimensional difference L₁: 40 mm
Dimensional difference L₂: 40 mm
Dimensional difference L₃: 40 mm
Dimensional difference L₄: 40 mm
Gas flow velocity within the baffle plate: 15.4 m/s
Particle diameter of sprayed liquid drops: 550 µm

### [Comparative Example 2]

Distillation was performed in the same manner except that in Example 1, the particle diameter of sprayed drops of the liquid containing a polymerization inhibitor was changed to 550 µm. As a result, the number of days for which the operation can be stably continued was 30 days.

This application is based on Japanese Patent Application (Patent Application No. 2018-007446) filed on January 19, 2018 and Japanese Patent Application (Patent Application No. 2019-003553) filed on January 11, 2019.

### REFERENCE SIGNS LIST

1: Distillation column
2: Regular filler
3: Irregular filler
4: Plates
5: Feed line
6: Column top gas line
7: Reflux tank
8: Reflux line
9: Remainder line
10: Circulation line
11: Reboiler
12: Withdrawal line
17: Circulation line
18: Polymerization inhibitor feed line
20: Vertical multi-tube heat exchanger
21: Tubular body
22A: Inflow port
22B: Outflow port
21a: Outflow port
21b: Inflow port
22a: Upper lid section
22b: Lower lid section
23: Heat Transfer tube
24a: Upper tube plate
24b: Lower tube plate
25: Receiving chamber
26: Extraction chamber
27, 27', 27": Baffle plate
27A: Vertical plate section
27B: Top plate section
27C: First side plate section
27D: Second side plate section
27E: Extension strip
27T: Relay space
27f: Frame
27m, 27n: Small opening
28: Atomizing nozzle
30: Steam ejector
31: Suction pipe
31A: Junction port
31B: Circle
32: Steam pipe
33: Condenser
34: Discharge pipe
35: Pipe
36: Hot well tank
37: Pressure control valve
38: Pipe
P: Horizontal plane

## Claims

1. A production method of a (meth)acrylic acid or an ester thereof, comprising a step of, under a reduced pressure created by a pressure reducing device, forming a distillation gas from a process liquid containing a (meth)acrylic acid or an ester thereof and allowing the distillation gas to turn into a condensate through a vertical multi-tube heat exchanger (20), wherein
the vertical multi-tube heat exchanger (20) comprises
a tubular body (21),
an upper tube plate (24a) and a lower tube plate (24b) arranged respectively on an upper end side and a lower end side of the tubular body (21),
a plurality of heat transfer tubes (23) erected between the upper tube plate (24a) and the lower tube plate (24b),
an upper lid section (22a) provided on an upper side of the upper tube plate (24a),
a lower lid section (22b) provided on a lower side of the lower tube plate (24b),
a receiving chamber (25) surrounded by the upper tube plate (24a) and the upper lid section (22a),
an extraction chamber (26) surrounded by the lower tube plate (24b) and the lower lid section (22b),
a junction port (31A) provided in the lower lid section (22b), to which a suction pipe (31) connecting the vertical multi-tube heat exchanger (20) and the pressure reducing device is joined, and
a baffle plate (27, 27', 27") provided in the extraction chamber (26) and having a vertical plate section (27A) facing the junction port (31A), and
the distillation gas and a liquid containing a polymerization inhibitor are introduced into the receiving chamber (25), the distillation gas is cooled in the course of flowing inside the heat transfer tube (23) to produce a condensate, and the condensate and uncondensed distillation gas flow into the extraction chamber (26),
an area of the vertical plate section (27A) of the baffle plate (27, 27', 27") is larger than an opening area of the junction port (31A),
a top plate section (27B) is provided to connect the upper edge of the vertical plate section (27A) and an inner wall of the lower lid section (22b),
the uncondensed distillation gas within the extraction chamber (26) flows into a relay space (27T) surrounded by the baffle plate (27, 27', 27") and the inner wall of the lower lid section (22b) and flows out to the suction pipe (31) from the relay space (27T) via the junction port (31A),
the liquid containing a polymerization inhibitor is sprayed as drops from an atomizing nozzle (28) into the receiving chamber (25),
an atomizing nozzle (28) where when water is sprayed from the atomizing nozzle (28) under the same conditions as in the case of spraying the liquid containing a polymerization inhibitor from the atomizing nozzle (28) into the receiving chamber (25), the Sauter mean diameter of water drops sprayed becomes from 570 to 1,500 µm is used as the atomizing nozzle (28), and
an average gas flow velocity of the uncondensed distillation gas flowing into the relay space (27T) from the extraction chamber (26) is 15.0 m/s or less.

2. The production method of a (meth)acrylic acid or an ester thereof according to claim 1, wherein
the vertical plate section (27A) is provided substantially in parallel to the junction port (31A);
in the baffle plate (27, 27', 27"),
a first side plate section (27C) connecting one side edge of the vertical plate section (27A) and the inner wall of the lower lid section (22b) and being joined to one side edge of the top plate section (27B), and
a second side plate section (27D) connecting another side edge of the vertical plate section (27A) and the inner wall of the lower lid section (22b) and being joined to another side edge of the top plate section (27B), are provided; and
the baffle plate (27, 27', 27") is configured such that the uncondensed distillation gas flows from the baffle plate's (27, 27', 27") lower surface side into the relay space (27T).

3. The production method of a (meth)acrylic acid or an ester thereof according to claim 2, wherein when the junction port (31A) is projected in a direction perpendicular to the inner wall of the lower lid section (22b) onto the vertical plate section (27A), the shortest distance between an outer circumference of a projected image and an outer circumference of the vertical plate section (27A) is 50 mm or more.

4. The production method of a (meth)acrylic acid or an ester thereof according to claim 2 or 3, wherein a small opening is provided in at least one of the first side plate section (27C) and the second side plate section (27D) and the uncondensed distillation gas flows also from the small opening into the relay space (27T).

5. The production method of a (meth)acrylic acid or an ester thereof according to any one of claims 1 to 4, wherein an extension strip (27E) hanging from the lower edge of the vertical plate section (27A) and extending with inclining to approach toward the lower lid section (22b) on the lower side of the junction port (31A) is provided.

6. The production method of a (meth)acrylic acid or an ester thereof according to any one of claims 1 to 5, wherein the baffle plate (27, 27', 27") is configured such that the uncondensed distillation gas flows from the baffle plate's (27, 27', 27") lower surface portion and both side surface portions into the relay space (27T).

7. The production method of a (meth)acrylic acid or an ester thereof according to any one of claims 1 to 6, wherein the lower lid section (22b) has a substantially vertical surface and the junction port (31A) is provided in the substantially vertical surface.

8. The production method of a (meth)acrylic acid or an ester thereof according to any one of claims 1 to 7, wherein the pressure reducing device is a steam ejector (30).

9. The production method of a (meth)acrylic acid or an ester thereof according to any one of claims 1 to 8, wherein a downwardly inclined section toward the junction port (31A) is provided in the suction pipe (31).

10. The production method of a (meth)acrylic acid or an ester thereof according to any one of claims 1 to 9, wherein the exterior surface of the suction pipe (31) is kept warm with a heat-insulating material.

11. The production method of a (meth)acrylic acid or an ester thereof according to any one of claims 1 to 10, wherein the exterior surface of the suction pipe (31) is warmed by steam tracing or electric tracing.

## Patentansprüche

1. Verfahren zur Herstellung einer (Meth)acrylsäure oder eines Esters davon, umfassend einen Schritt des Bildens eines Destillationsgases aus einer Prozessflüssigkeit, die eine (Meth)acrylsäure oder einen Ester davon enthält, unter einem reduzierten Druck, der durch eine Druckreduziervorrichtung erzeugt wird, und des Erlaubens, dass sich das Destillationsgas durch einen vertikalen Mehrröhren-Wärmetauscher (20) in ein Kondensat verwandelt, wobei
der vertikale Mehrröhren-Wärmetauscher (20) Folgendes umfasst einen rohrförmigen Körper (21),
eine obere Rohrplatte (24a) und eine untere Rohrplatte (24b), die jeweils an einer oberen und einer unteren Endseite des rohrförmigen Körpers (21) angeordnet sind,
eine Vielzahl von Wärmeübertragungsrohren (23), die zwischen der oberen Rohrplatte (24a) und der unteren Rohrplatte (24b) angeordnet sind,
einen oberen Deckelteil (22a), der an einer Oberseite der oberen Rohrplatte (24a) bereitgestellt ist,
einen unteren Deckelteil (22b), der an einer Unterseite der unteren Rohrplatte (24b) bereitgestellt ist,
eine Aufnahmekammer (25), die von der oberen Rohrplatte (24a) und dem oberen Deckelteil (22a) umgeben ist,
eine Extraktionskammer (26), die von der unteren Rohrplatte (24b) und dem unteren Deckelteil (22b) umgeben ist,
ein in dem unteren Deckelteil (22b) bereitgestellter Abzweigport (31 A), an den eine den vertikalen Mehrrohrwärmetauscher (20) und die Druckreduziervorrichtung verbindende Saugleitung (31) angeschlossen ist, und
eine Prallplatte (27, 27', 27"), die in der Extraktionskammer (26) bereitgestellt ist und einen vertikalen Plattenteil (27A) aufweist, der dem Abzweigport (31 A) zugewandt ist, und
wobei das Destillationsgas und eine Flüssigkeit, die einen Polymerisationsinhibitor enthält, in die Aufnahmekammer (25) eingeleitet werden, das Destillationsgas im Verlauf der Strömung innerhalb des Wärmeübertragungsrohrs (23) abgekühlt wird, um ein Kondensat herzustellen, und das Kondensat und das unkondensierte Destillationsgas in die Extraktionskammer (26) strömen,
eine Fläche des vertikalen Plattenteils (27A) der Prallplatte (27, 27', 27") größer ist als eine Öffnungsfläche des Abzweigports (31A),
ein oberer Plattenteil (27B) bereitgestellt ist, um den oberen Rand des vertikalen Plattenteils (27A) und eine Innenwand des unteren Deckelteils (22b) zu verbinden,
das unkondensierte Destillationsgas innerhalb der Extraktionskammer (26) in einen Relaisraum (27T) strömt, der von der Prallplatte (27, 27', 27") und der Innenwand des unteren Deckelteils (22b) umgeben ist, und aus dem Relaisraum (27T) über den Abzweigport (31A) zu dem Saugrohr (31) strömt,
die einen Polymerisationsinhibitor enthaltende Flüssigkeit als Tropfen aus einer Zerstäuberdüse (28) in die Aufnahmekammer (25) gesprüht wird,
als Zerstäubungsdüse (28) eine Zerstäubungsdüse (28) verwendet wird, bei der, wenn Wasser aus der Zerstäubungsdüse (28) unter den gleichen Bedingungen wie beim Sprühen der einen Polymerisationsinhibitor enthaltenden Flüssigkeit aus der Zerstäubungsdüse (28) in die Aufnahmekammer (25) gesprüht wird, der mittlere Sauter-Durchmesser der gesprühten Wassertropfen 570 bis 1.500 pm beträgt, und
eine durchschnittliche Gasströmungsgeschwindigkeit des unkondensierten Destillationsgases, das aus der Extraktionskammer (26) in den Relaisraum (27T) strömt, 15,0 m/s oder weniger beträgt.

2. Verfahren zur Herstellung einer (Meth)acrylsäure oder eines Esters davon nach Anspruch 1, wobei
der vertikale Plattenteil (27A) im Wesentlichen parallel zu dem Abzweigport (31A) bereitgestellt ist;
in der Prallplatte (27, 27', 27")
ein erster Seitenplattenteil (27C) eine Seitenkante des vertikalen Plattenteils (27 A) und die Innenwand des unteren Deckelteils (22b) verbindet und mit einer Seitenkante des oberen Plattenabschnitts (27B) verbunden ist, und
ein zweiter Seitenplattenteil (27D) bereitgestellt ist, der eine andere Seitenkante des vertikalen Plattenteils (27A) und die Innenwand des unteren Deckelteils (22b) verbindet und mit einer anderen Seitenkante des oberen Plattenteils (27B) verbunden ist; und
die Prallplatte (27, 27', 27") so konfiguriert ist, dass das unkondensierte Destillationsgas von der Unterseite der Prallplatte (27, 27', 27") in den Relaisraum (27T) strömt.

3. Verfahren zur Herstellung einer (Meth)acrylsäure oder eines Esters davon nach Anspruch 2, wobei, wenn der Abzweigport (31 A) in einer Richtung senkrecht zu der Innenwand des unteren Deckelteils (22b) auf den vertikalen Plattenteil (27A) projiziert wird, der kürzeste Abstand zwischen einem Außenumfang eines projizierten Bildes und einem Außenumfang des vertikalen Plattenteils (27A) 50 mm oder mehr beträgt.

4. Verfahren zur Herstellung einer (Meth)acrylsäure oder eines Esters davon nach Anspruch 2 oder 3, wobei eine kleine Öffnung in mindestens einem der ersten Seitenplattenteile (27C) und des zweiten Seitenplattenteils (27D) bereitgestellt ist und das nicht kondensierte Destillationsgas auch aus der kleinen Öffnung in den Relaisraum (27T) strömt.

5. Verfahren zur Herstellung einer (Meth)acrylsäure oder eines Esters davon nach einem der Ansprüche 1 bis 4, wobei ein Verlängerungsstreifen (27E) bereitgestellt ist, der von der Unterkante des vertikalen Plattenteils (27A) herabhängt und sich mit einer Neigung zur Annäherung an den unteren Deckelteil (22b) auf der Unterseite des Abzweigports (31A) erstreckt.

6. Verfahren zur Herstellung einer (Meth)acrylsäure oder eines Esters davon nach einem der Ansprüche 1 bis 5, wobei die Prallplatte (27, 27', 27") so konfiguriert ist, dass das unkondensierte Destillationsgas von dem unteren Oberflächenabschnitt der Prallplatte (27, 27', 27") und den beiden seitlichen Oberflächenabschnitten in den Relaisraum (27T) strömt.

7. Verfahren zur Herstellung einer (Meth)acrylsäure oder eines Esters davon nach einem der Ansprüche 1 bis 6, wobei der untere Deckelteil (22b) eine im wesentlichen vertikale Oberfläche aufweist und der Abzweigport (31 A) in der im wesentlichen vertikalen Oberfläche bereitgestellt ist.

8. Verfahren zur Herstellung einer (Meth)acrylsäure oder eines Esters davon nach einem der Ansprüche 1 bis 7, **dadurch gekennzeichnet, dass** die Druckminderungsvorrichtung ein Dampfejektor (30) ist.

9. Verfahren zur Herstellung einer (Meth)acrylsäure oder eines Esters davon nach einem der Ansprüche 1 bis 8, **dadurch gekennzeichnet, dass** in dem Saugrohr (31) ein zu dem Abzweigport (31 A) hin abwärts geneigter Teil bereitgestellt ist.

10. Verfahren zur Herstellung einer (Meth)acrylsäure oder eines Esters davon nach einem der Ansprüche 1 bis 9, wobei die Außenfläche des Saugrohrs (31) mit einem wärmeisolierenden Material warmgehalten wird.

11. Verfahren zur Herstellung einer (Meth)acrylsäure oder eines Esters davon nach einem der Ansprüche 1 bis 10, wobei die Außenfläche des Saugrohrs (31) durch Dampfbeheizung oder elektrische Beheizung erwärmt wird.

## Revendications

1. Procédé de production d'un acide (méth)acrylique ou d'un ester de celui-ci, comprenant une étape consistant, sous une pression réduite créée par un dispositif de réduction de pression, à former un gaz de distillation à partir d'un liquide de procédé contenant un acide (méth)acrylique ou un ester de celui-ci et à permettre au gaz de distillation de se transformer en un condensat à travers un échangeur de chaleur multitube vertical (20), dans lequel
l'échangeur de chaleur multitube vertical (20) comprend un corps tubulaire (21),
une plaque de tube supérieure (24a) et une plaque de tube inférieure (24b) agencées respectivement sur un côté d'extrémité supérieure et un côté d'extrémité inférieure du corps tubulaire (21),
une pluralité de tubes de transfert de chaleur (23) érigés entre la plaque de tube supérieure (24a) et la plaque de tube inférieure (24b),
une section de couvercle supérieure (22a) fournie sur un côté supérieur de la plaque de tube supérieure (24a),
une section de couvercle inférieure (22b) fournie sur un côté inférieur de la plaque de tube inférieure (24b),
une chambre de réception (25) entourée par la plaque de tube supérieure (24a) et la section de couvercle supérieure (22a),
une chambre d'extraction (26) entourée par la plaque de tube inférieure (24b) et la section de couvercle inférieure (22b),
un orifice de jonction (31A) fourni dans la section de couvercle inférieure (22b), auquel est raccordé un tuyau d'aspiration (31) raccordant l'échangeur de chaleur multitube vertical (20) et le dispositif de réduction de pression, et
une plaque de déflecteur (27, 27', 27") fournie dans la chambre d'extraction (26) et présentant une section de plaque verticale (27A) faisant face à l'orifice de jonction (31A), et
le gaz de distillation et un liquide contenant un inhibiteur de polymérisation sont introduits dans la chambre de réception (25), le gaz de distillation est refroidi au cours de son écoulement à l'intérieur du tube de transfert de chaleur (23) pour produire un condensat, et le condensat et le gaz de distillation non condensé s'écoulent dans la chambre d'extraction (26),
une zone de la section de plaque verticale (27A) de la plaque de déflecteur (27, 27', 27") est plus grande qu'une zone d'ouverture de l'orifice de jonction (31A),
une section de plaque supérieure (27B) est fournie pour relier le bord supérieur de la section de plaque verticale (27A) et une paroi interne de la section de couvercle inférieure (22b),
le gaz de distillation non condensé dans la chambre d'extraction (26) s'écoule dans un espace de relais (27T) entouré par la plaque de déflecteur (27, 27', 27") et la paroi interne de la section de couvercle inférieure (22b) et s'écoule vers le tuyau d'aspiration (31) à partir de l'espace de relais (27T) via l'orifice de jonction (31A),
le liquide contenant un inhibiteur de polymérisation est pulvérisé sous forme de gouttes à partir d'une
buse d'atomisation (28) dans la chambre de réception (25),
une buse d'atomisation (28) avec laquelle, quand de l'eau est pulvérisée à partir de la buse d'atomisation (28) dans les mêmes conditions que dans le cas de la pulvérisation du liquide contenant un inhibiteur de polymérisation à partir de la buse d'atomisation (28) dans la chambre de réception (25), le diamètre moyen de Sauter des gouttes d'eau pulvérisées est de 570 à 1500 µm est utilisée comme buse d'atomisation (28), et
la vitesse moyenne d'écoulement de gaz du gaz de distillation non condensé s'écoulant dans l'espace de relais (27T) à partir de la chambre d'extraction (26) est de 15,0 m/s ou moins.

2. Procédé de production d'un acide (méth)acrylique ou d'un ester de celui-ci selon la revendication 1, dans lequel
la section de plaque verticale (27A) est fournie sensiblement parallèlement à l'orifice de jonction (31A) ;
dans la plaque de déflecteur (27, 27', 27"),
une première section de plaque latérale (27C) reliant un bord latéral de la section de plaque verticale (27A) et la paroi interne de la section de couvercle inférieure (22b) et étant jointe à un bord latéral de la section de plaque supérieure (27B), et
une seconde section de plaque latérale (27D) reliant un autre bord latéral de la section de plaque verticale (27A) et la paroi interne de la section de couvercle inférieure (22b) et étant jointe à un autre bord latéral de la section de plaque supérieure (27B), sont fournies ; et
la plaque de déflecteur (27, 27', 27") est configurée de sorte que le gaz de distillation non condensé s'écoule à partir du côté de surface inférieure de la plaque de déflecteur (27, 27', 27") dans l'espace de relais (27T).

3. Procédé de production d'un acide (méth)acrylique ou d'un ester de celui-ci selon la revendication 2, dans lequel quand l'orifice de jonction (31A) est projeté dans une direction perpendiculaire à la paroi interne de la section de couvercle inférieure (22b) sur la section de plaque verticale (27A), la distance la plus courte entre une circonférence externe d'une image projetée et une circonférence externe de la section de plaque verticale (27A) est de 50 mm ou plus.

4. Procédé de production d'un acide (méth)acrylique ou d'un ester de celui-ci selon la revendication 2 ou 3, dans lequel une petite ouverture est fournie dans au moins l'une parmi la première section de plaque latérale (27C) et la seconde section de plaque latérale (27D) et le gaz de distillation non condensé s'écoule également à partir de la petite ouverture dans l'espace de relais (27T).

5. Procédé de production d'un acide (méth)acrylique ou d'un ester de celui-ci selon l'une quelconque des revendications 1 à 4, dans lequel une bande d'extension (27E) suspendue à partir du bord inférieur de la section de plaque verticale (27A) et s'étendant avec une inclinaison pour se rapprocher de la section de couvercle inférieure (22b) sur le côté inférieur de l'orifice de jonction (31A) est fournie.

6. Procédé de production d'un acide (méth)acrylique ou d'un ester de celui-ci selon l'une quelconque des revendications 1 à 5, dans lequel la plaque de déflecteur (27, 27', 27") est configurée de telle sorte que le gaz de distillation non condensé s'écoule à partir de la partie de surface inférieure de la plaque de déflecteur (27, 27', 27") et des deux parties de surface latérale dans l'espace de relais (27T).

7. Procédé de production d'un acide (méth)acrylique ou d'un ester de celui-ci selon l'une quelconque des revendications 1 à 6, dans lequel la section de couvercle inférieure (22b) présente une surface sensiblement verticale et l'orifice de jonction (31A) est fourni dans la surface sensiblement verticale.

8. Procédé de production d'un acide (méth)acrylique ou d'un ester de celui-ci selon l'une quelconque des revendications 1 à 7, dans lequel le dispositif de réduction de pression est un éjecteur à vapeur d'eau (30).

9. Procédé de production d'un acide (méth)acrylique ou d'un ester de celui-ci selon l'une quelconque des revendications 1 à 8, dans lequel une section inclinée vers le bas vers l'orifice de jonction (31A) est fournie dans le tuyau d'aspiration (31).

10. Procédé de production d'un acide (méth)acrylique ou d'un ester de celui-ci selon l'une quelconque des revendications 1 à 9, dans lequel la surface externe du tuyau d'aspiration (31) est maintenue chaude avec un matériau isolant thermique.

11. Procédé de production d'un acide (méth)acrylique ou d'un ester de celui-ci selon l'une quelconque des revendications 1 à 10, dans lequel la surface externe du tuyau d'aspiration (31) est chauffée par traçage à la vapeur ou traçage électrique.
